# EUROPEAN PATENT SPECIFICATION

(11) **EP 0 500 632 B1**
(45) Date of publication and mention of the grant of the patent: **08.04.1998**
(21) Application number: 90916436.0
(22) Date of filing: 09.11.1990
(51) Int. Cl.: A61F 5/14, A43B 7/22, A43B 13/38

(54) **METHOD OF FORMING ORTHOTIC DEVICES**
VERFAHREN ZUR FORMGEBUNG ORTHOTISCHER VORRICHTUNGEN
METHODE POUR FORMER DES DISPOSITIFS ORTHOTIQUES

(30) Priority: 17.11.1989 AU PJ7446/89
(43) Date of publication of application: 02.09.1992
(60) Divisional application: 97202791.6
(73) Proprietor: FREEHAVEN INVESTMENTS LTD, Charlestown, Nevis (KN)
(72) Inventor: VASYLI, Phillip, John, Kirrawee, NSW 2232 (AU)
(74) Representative: Findlay, Alice Rosemary
(86) International application number: AU9000543
(87) International publication number: WO9107152

(56) References cited:
- EP-A- 0 118 319
- AU-A- 4 063 085
- AU-B- 1 231 343
- AU-B- 7 948 382
- DE-U- 8 700 681
- GB-A- 1 066 996
- US-A- 2 313 870
- US-A- 2 401 514
- US-A- 3 895 405
- US-A- 4 216 778
- US-A- 4 232 457
- US-A- 4 333 472

## Description

### TECHNICAL FIELD

This invention relates to orthotic devices and more particularly to such a device which is able to be molded to a patient's foot, "in situ" in an article of footwear, to give support to, and to control, the osseous structures of the foot.

### BACKGROUND ART

So called "orthotic devices" are known, ranging from simple contoured insoles to costly structures integrally incorporated into made-to-order orthopaedic footwear.

Orthopaedic footwear apart, known insertable insole devices as a general rule overlie the whole of the upper surface of the liner of the shoe sole, thus requiring the existence of an extensive range of lengths, widths and even shapes - for example, the court-fit shape in women's dress shoes. These known insoles are usually sold "off the shelf" and provide but indifferent biomechanical control or if they are fitted to the patient's shoe, the fitting and adjustment time may be quite long. They rarely provide satisfactory bio-mechanical control for, and control of, the osseous structures of the foot and are often made of unyielding materials such as hard plastic or carbon fibre; on the other hand, many resilient molded insoles merely function as shock absorbers.

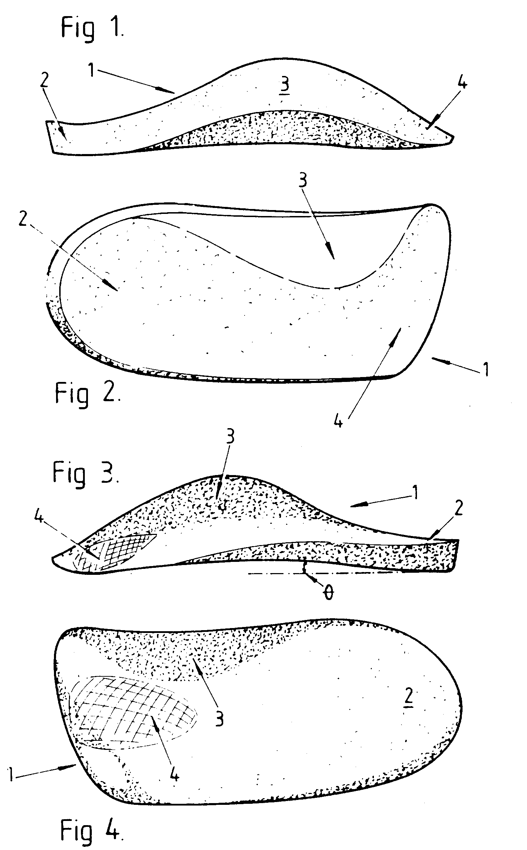

"Full length" orthotic devices are disclosed in such U.S. Patents as No. 3,895,405 (EDWARDS) in which insoles are molded from an initially flat sheet (see Figures 1 and 2) by a method comprising the steps of inserting the device inside an article of footwear, positioning the device so as to overlie part of the upper surface of the sole, heating the device with a stream of heated air so as to render the upper contour mouldable, fitting the user's foot into the article of footwear and allowing the device to cool; in No. 3,782,390 (JOHNSON) is taught a method of producing inserts by injecting a curable fluid into a shoe 12 in which is a foot 8; No. 2,760,281 (COSIN) discloses an insole or insert composed of several discrete layers including a "sandwiched" triangular element 15; and No. 2,409,594 (SHERMAN) discloses a corrective foot appliance formed from sheet material which is "capable of becoming relatively permanently set in shape in the course of time when treated with an activator.".

U.S. Patent Nos. 4,674,204; 4,232,457; 3,825,017 and 4,522,777, to SULLIVAN, MOSHER, SCRIMA and PETERSON respectively disclose various orthotic insoles having spongy or foam layers.

In U.S. Patent No. 2,401,514 (SCHOLL) the inventive concept is a strip of fabric 15 applied to the underside of a thermoplastic resin arch support to prevent it from sliding and squeaking when worn inside a shoe; U.S. Patent No. 4,517,981 (SANTOPIETRO) relates to a substantially flat, three-quarter length orthotic device having no longitudinal arch "raise" or metatarsal "raise". U.S. Patent No. 4,823,420 (BARTNECK) discloses a contour molded insole, including several layers of material; it is apparently somewhat less than three-quarter length and it is abitrarily cut off in a straight transverse front edge provided with no metatarsal "raise".

Other specifications of interest are U.S. Patent Nos 2,653,396 (GOTTLIEB); 3,068,872 (BRODY); 3,121,431 (ROSENHAFT); 3,859,740 (KEMP); 3,309,797 (POATIS); 4,216,778 (WEISS); 4,268,980 (GUDAS); 4,346,525 (LARSEN); 4,364,188 (TURNER); 4,463,761 (POIS), 4,520,581 (IRWIN); 4,530,173 (GESINSKY); 4,557,060 (KAWASHIMA); 4,563,787 (DREW); 4,674,201 (WEISS); 4,686,993 (GRUMBINE); 4,702,255 (SCHENKI); 4,756,096 (MAYER); 4,791,736 (PHILLIPS) AND 4,868,945 (DE VETTIGNIS).

British Patent 1066996 describes an arch support device which molds to the surface of a wearer's foot under the pressure of the wearer's weight, the material and shape being such that the device is restored to its original shape on removal of body pressure. The device as supplied may include an elevation to give support to the metatarsal or transverse arch of the foot and a heel seat. It is, however, completely reshapable under heating by placement in a pan of hot water. The device functions primarily, due to its deformable nature, as a cushioning arch support rather than a corrective orthotic.

EP-A-0154170 discloses an arrangement for molding an insole from an essentially formless pad. The molded features are essentially simply a cast of the patient's foot.

It is an object of the present invention to overcome or, at least, mitigate certain disadvantages and shortcomings of the prior art.

In accordance with the invention there is provided a method of forming a customized orthotic device for insertion in an article of footwear using a pre-contoured mouldable orthotic device, the device being formed with pre-determined orthotic structures, said orthotic structures including an integrally moulded heel cup, a longitudinal arch raise, a metatarsal raise for aligning the heads of the second, third and fourth metatarsal bones, and a varus post of about 4 degrees, the device being of a length so as to operatively position the heel cup to underlie the heel bone and the metatarsal rise to underlie the head ends of the second, third and fourth metatarsal bones of said user's foot, and the device is formed of a resilient material which however is such that the upper contour of the orthotic device is adapted to be altered by heating the device, the method comprising the steps of:-
(a) inserting the device inside an article of footwear
(b) positioning the device as far to the rear of the article of footwear as possible, so as to overlie part of the upper surface of the sole
(c) heating the device with a stream of heated air so as to render the upper contour mouldable whilst substantially retaining said orthotic structures
(d) fitting the user's foot into the article of footwear with the user seated
(e) palpating the user's subtalar joint to a neutral position
(f) with the user standing putting equal weight on each foot while maintaining the neutral position of the sub-talar joint, pressing medial and lateral edges of the article of footwear inwards so as to contour the now mouldable orthotic device to the user's neutral foot position, and
(g) allowing the device to cool.

The contoured moldable orthotic device having certain pre-determined shapes and angles and is adapted to be inserted into an article of footwear so as to overlie part of the upper surface of a sole thereof and to be able to subsequently molded in situ to a patient's foot to thereby give support to, and to control, the osseous structure thereof. The length of said moldable orthotic device is such as to underlie the heel bone and to terminate adjacent the head ends of the metatarsal of a said patient's foot; said orthotic device being formed with an integrally molded heel cup, a longitudinal arch raise, a varus post of about 4°, and a metatarsal raise for aligning the heads of the second, third and fourth metatarsals.

Ideally, the width of the moldable orthotic device is that distance from the lateral aspect of the head of the fifth metatarsal to the longitudinal bisection of the first and second metatarsals; the arrangement being such that the shaft of the first metatarsal is able to plantarflex during the propulsive phase of a said patient's foot. The length is substantially three-quarters of the length of the article of footwear.

The terms "raise" and "plantarflex" are well-understood by those familiar with the field of orthotic devices.

The device is heated, preferably in a sequence cycle of about five seconds in an "on" mode followed by about five seconds in an "off" mode with the cycle repeated until such time as said device has undergone a total heating time of about twenty seconds;

### BRIEF DESCRIPTION OF THE DRAWINGS

In order that a better understanding of the present invention may be gained, hereinafter will be described preferred embodiments thereof, by way of example only and with reference to the accompanying drawings in which:-
Figure 1 is a side elevation of the right-hand side of a left-foot moldable orthotic device;
Figure 2 is a bottom plan view;
Figure 3 is a side elevation of the left-hand side of the device;
Figure 4 is a top plan view;
Figure 5 is a frontal top perspective;
Figure 6 is a fragmentary view of the bottom of the heel portion of another embodiment which is provided with a shock-absorbing, spongy "shock dot" insert; and
Figure 7 is a cross-section along line VII-VII of Figure 6, but somewhat exaggerated as regards proportions.

Throughout the drawings, like integers are referrenced by the same numeral and, throughout the specification, the adjective "orthotic" is used to qualify "device", instead of the alternatives "orthotic", "orthopodic" or "orthopaedic".

In the drawings there is shown a moldable orthotic device, generally referenced 1, which may well be of such a material as 350 Kg/M³ density "ETHYL VINYL ACETATE" (E.V.A.) or 220 Kg/M³ density "ETHYL VINYL ACETATE" (E.V.A.). The specifications of these two materials are as in the following table:-

| PROPERTY & TEST METHOD | UNITS | "E.V.A." | "E.V.A." |
|---|---|---|---|
| DENSITY | Kg/M³ | 350 | 220 |
| HARDNESS (jis type C) | - | 74 | 57 |
| WATER ABSORPTION (jis K6767) | gms/cm³ | <0.002 | <0.002 |
| THERMAL CONDUCTIVITY (astm 578) | W/M°C. | 0.064 | 0.055 |
| OPERATING TEMPERATURE | Min °C | -70°C | -70°C |
| | Max °C | 70°C | 70°C |
| TENSILE STRENGTH (jis K6767) | MPa | 3.0 | 2.0 |
| TEAR STRENGTH (jis K6767) | N/Cm | 170 | 120 |
| ELONGATION AT BREAK (jis K6767) | % | 250-300 | 250-300 |
| COMPRESSION SET | % | < 5 | < 5 |
| COMPRESSION DEFLECTION (astm d3575) | KPa | 950 | 240 |

The moldable orthotic device may be provided in various geometries, that is to say, in various shapes, as regular adult; women's "court fit"; infants; and youths. Children's size ranges envisaged are, say, 1 - 3; 4 - 6; 7 - 9; 10 - 12; and 13 youths' size 2; in regular and wide fittings: thus, ten 'models' are provided. Also envisaged are adult's sizes 4 - 6; 7 - 9; and 10 - 12; in regular, wide and "court" fittings, nine adult models being provided in this range.

Each moldable orthotic device 1 is formed of E.V.A. from a positive cast based on that same configuration as are known rigid devices. The length of the device is substantially three-quarters of the length of the inside of the article of footwear so as to underlie the heel bone, (the calcaneus or os calcis,), terminating adjacent the heads of the metatarsals, which are those bones between the phalangeal bones and the seven tarsal bones that articulate the foot.

Each orthotic device 1 is formed with an integrally molded heel cup 2, longitudinal arch "raise" 3, and a metatarsal raise 4 for the purpose of aligning the heads of the second, third and fourth metatarsals.

The width of the moldable orthotic device ideally is from the lateral aspect of the fifth metatarsal bone's head to the longitudinal bisection of the first and second metatarsals. This arrangement permits the shaft of the first metatarsal bone to "plantarflex" during the propulsive phase - that is to say, during walking or running. If necessary, additional wedging and/or other geometric configurations might well be incorporated for the purpose of correcting severe fore-foot, midtarsal and/or rear-foot deformities.

It is contemplated that, initially at least, specifically-trained technical personnel will "custom-mold" the inventive moldable orthotic devices onto existing pre-molded cast templates; however, it is envisaged that a long-term production process will combine in-house "cad/cam" manufacturing procedures with some utilisation of industrial molding techniques.

For certain foot malformations, moldable adjuncts may include 2°, 4°, 6°, or 8° angle adhesive wedges; 4 or 8 mm heel adhesive "raises" and fabric covers for the moldable orthotic devices.

A 4° - angled (or thereabouts) rear foot post - a so-called "varus post" - as indicated by the angle θ shown in Figure 3, is in-built to allow for leg curvature and to prevent excess pronation.

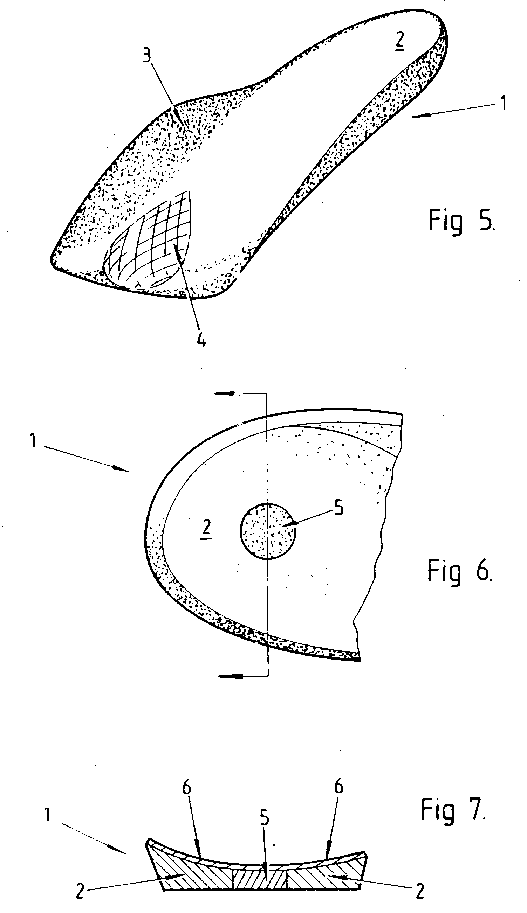

In a modification, the pre-molded orthotic device may incorporate a sponge-like, like shock-absorbing insert (highly preferably made of low-density polyurethane foam) 5 which is adapted to cushion that area immediately beneath the heel spur or calcaneus. Such an insert 5 may aptly be termed a "shock dot", or "shock spot". The cushioned area may extend either fully or partially through the full thickness of the orthotic device. Advantageously, the upper surface of the orthotic device may be covered, or sheathed, with a fabric-like outer "skin" - as referenced 6 in Figure 7.

The present invention comprises a method, for the "in situ" molding, to a patient's foot, of the moldable orthotic device described above after the device has been inserted into an article of footwear so as to overlie part of the upper surface of its sole.

The molding process is as follows:- A moldable orthotic device as described above is inserted into a selected article of footwear, placed as far back as it will go. The device is then heated with a stream of hot air, from a suitable source, using a sequence cycle of about five seconds "on" followed by about five seconds "off". This cycle is repeated until such time as the device has undergone a total heating time of about twenty seconds. The patient is seated and his or her foot is fitted into the boot or shoe; palpate the subtalar joint in the known manner to neutral position. The patient is then required to stand, putting equal weight on each foot, while maintaining the neutral position of the subtalar joint. The medial and lateral edges of the article of footwear are pressed inwards so as to contour the warm moldable orthotic device to the patient's neutral foot position. The device need only to be cooled for perhaps five minutes before the boot or shoe is ready for wearing, perhaps cooled to ambient temperature.

The procedure is then repeated for the other foot, if necessary; however, both articles of footwear of the pair should be worn to ensure equal balance during the molding of each device. If required, fore-foot posts might well be applied to the plantar distal orthotic device's edge subsequent to the molding process. Moreover, it will be realised that an moldable orthotic device may be easily re-molded if the desired result is not initially attained.

The present invention offers several distinct advantages over and above the prior art devices:- greater rear foot control action due to the high and solid heel cup: integrated cushioning "shock dot": correctly contoured for metatarsal correction, longitudinal arch correction, lateral arch correction and sagittal calcaneal correction: greater stability due to the basic solidity of E.V.A. and to the wide surface rear foot area: in-built varus post, ideally 4°.

Tests have shown that the inventive moldable orthotic device is well able to provide a remedy for common biomechanical problems relating to the foot as listed hereafter:- heel spurs: plantar fasciitis: Metatarsalgia: claw toes: calcaneal apophysitis: achilles tendonitis: shin splints: excess pronation: patella tracking malfunctions: flat feet: and like problems of an orthopaedic nature.

## Claims

1. A method of forming a customized orthotic device for insertion in an article of footwear using a pre-contoured mouldable orthotic device (1), the device being formed with pre-determined orthotic structures, said orthotic structures including an integrally moulded heel cup (2), a longitudinal arch raise (3), a metatarsal raise (4) for aligning the heads of the second, third and fourth metatarsal bones, and a varus post of about 4 degrees, the device being of a length so as to operatively position the heel cup to underlie the heel bone and the metatarsal rise to underlie the head ends of the second, third and fourth metatarsal bones of said user's foot, and the device is formed of a resilient material which however is such that the upper contour of the orthotic device is adapted to be altered by heating the device, the method comprising the steps of:-
(a) inserting the device inside an article of footwear
(b) positioning the device as far to the rear of the article of footwear as possible, so as to overlie part of the upper surface of the sole
(c) heating the device with a stream of heated air so as to render the upper contour mouldable whilst substantially retaining said orthotic structures
(d) fitting the user's foot into the article of footwear with the user seated
(e) palpating the user's subtalar joint to a neutral position
(f) with the user standing putting equal weight on each foot while maintaining the neutral position of the sub-talar joint, pressing medial and lateral edges of the article of footwear inwards so as to contour the now mouldable orthotic device to the user's neutral foot position, and
(g) allowing the device to cool.

2. A method according to Claim 1, wherein the stream of heated air is applied in a sequence cycle of about 5 seconds in an on mode followed by about 5 seconds in an off mode, and repeating the cycle until such time as the device has undergone a total heating cycle of about 20 seconds.

3. A method according to either Claim 1 or Claim 2, wherein after step (f), fore-foot and/or rear foot posts are applied to the plantar distal edge of the device.

4. A method as claimed in any one of Claims 1 to 3, comprising incorporating a shock-absorbing insert (5) in the device (1) adapted to cushion that area of a patient's foot which is immediately beneath the heel spur thereof.

5. A method as claimed in Claim 4, comprising extending the cushioned area either partially or fully through the full thickness of the device (1).

6. A method as claimed in any one of Claims 1 to 5, comprising sheathing the upper surface of said device (1) in a fabric-like outer skin (6).

7. A method as claimed in any one of Claims 1 to 6, comprising farming the device from ethyl vinyl acetate.

## Patentansprüche

1. Ein Verfahren zum Formen einer kundengerecht angepaßten orthotischen Vorrichtung zum Einsetzen in einen Schuhwerkartikel unter Verwendung einer vorkontourierten, formbaren orthotischen Vorrichtung (1), wobei die Vorrichtung mit vorbestimmten orthotischen Strukturen geformt wird und die besagten orthotischen Strukturen eine integrierend geformte Fersenmulde (2), eine Längserhöhung für das Quergewölbe (3), eine Metatarsalerhöhung (4) zum Ausrichten der Vorderenden des zweiten, des dritten und des vierten Metatarsalknochens des Fußes des Benutzers und eine in einem Winkel von etwa vier Graden verlaufende Varusstütze umfassen, die Länge der Vorrichtung so beschaffen ist, daß die Fersenmulde im Gebrauch unterhalb des Fersenbeins und die Metatarsalerhöhung unterhalb der Vorderenden des zweiten, des dritten und des vierten Metatarsalknochens des Fußes des Benutzers zu liegen kommen und die Vorrichtung aus einem elastischen Material geformt ist, das jedoch so beschaffen ist, daß die obere Kontour der orthotischen Vorrichtung durch Erhitzen der Vorrichtung abgeändert werden kann, und zwar umfaßt das Verfahren die folgenden Schritte:
(a) Einsetzen der Vorrichtung in einen Schuhwerkartikel.
(b) Anordnen der Vorrichtung möglichst weit hinten in dem Schuhwerkartikel, so daß sie einen Teil der oberen Sohlenoberfläche überdeckt.
(c) Erhitzen der Vorrichtung durch einen Strom erhitzter Luft, um die obere Kontour formbar zu machen, während gleichzeitig die besagten orthotischen Strukturen im wesentlichen beibehalten werden.
(d) Einpassen des Fußes des Benutzers in den Schuhwerkartikel, während der Benutzer sitzt.
(e) Palpieren des subtalaren Gelenks des Benutzers in eine neutrale Lage.
(f) Indem der Benutzer stehend unter Beibehaltung der neutralen Lage des subtalaren Gelenks auf jeden Fuß das gleiche Gewicht ausübt, Einwärtspressen der medialen und seitlichen Ränder des Schuhwerkartikels, um die nun formbare orthotische Vorrichtung der neutralen Fußlage des Benutzers entsprechend zu kontourieren, und
(g) Abkühlen lassen der Vorrichtung.

2. Ein Verfahren nach Anspruch 1, bei dem der Strom erhitzter Luft in einem Folgezyklus zur Einwirkung gebracht wird, wobei in dem besagten Zyklus auf einen Einschaltmodus von etwa 5 Sekunden Dauer ein Ausschaltmodus von etwa 5 Sekunden Dauer folgt, und zwar wird der besagte Zyklus so lange wiederholt, bis die Vorrichtung einen Erhitzungszyklus von insgesamt etwa 20 Sekunden Dauer durchgemacht hat.

3. Ein Verfahren nach Anspruch 1 oder Anspruch 2, bei dem nach Schritt (f) Vorderfuß- und/oder Hinterfußstützen an dem plantaren Distalrand der Vorrichtung angebracht werden.

4. Ein Verfahren nach einem der Ansprüche 1 bis 3, umfassend Einbeziehung eines stoßdämpfenden Einsatzes (5) in die Vorrichtung (1), der so beschaffen ist, daß er die unmittelbar unterhalb des Fersenbeinsporns befindliche Fläche des Fußes eines Patienten abfedert.

5. Ein Verfahren nach Anspruch 4, umfassend Erweiterung der abgefederten Fläche teilweise oder ganz durch die gesamte Dicke der Vorrichtung (1).

6. Ein Verfahren nach einem der Ansprüche 1 bis 5, umfassend Verkleidung der oberen Oberfläche der besagten Vorrichtung (1) mit einer gewebeartigen Außenhaut (6).

7. Ein Verfahren nach einem der Ansprüche 1 bis 6, umfassend Herstellung der Vorrichtung aus Äthylvinylacetat.

## Revendications

1. Procédé de formage d'un dispositif orthostatique personnalisé s'insérant dans une chaussure par utilisation d'un dispositif orthostatique moulable pré-profilé (1), le dispositif étant formé avec des structures orthostatiques prédéterminées, lesdites structures orthostatiques comprenant une cuvette moulée intégrée pour le talon (2), un support plantaire longitudinal (3), un support métatarsien (4) pour aligner les têtes des deuxième, troisième et quatrième os métatarsiens, et un montant varus d'environ 4 degrés, le dispositif étant d'une longueur propre à positionner, en utilisation, la cuvette pour le talon sous l'os du talon et le support métatarsien sous les extrémités des têtes des deuxième, troisième et quatrième os métatarsiens dudit pied de l'utilisateur, et le dispositif étant formé d'un matériau élastique qui est cependant tel que le contour supérieur du dispositif orthostatique est adapté pour être modifié par chauffage du dispositif, le procédé comprenant les étapes suivantes :
(a) insertion du dispositif à l'intérieur d'une chaussure
(b) positionnement du dispositif aussi loin en arrière que possible dans la chaussure, de manière à recouvrir une partie de la surface supérieure de la semelle
(c) chauffage du dispositif avec un courant d'air chauffé de manière à rendre le contour supérieur moulable tout en retenant substantiellement lesdites structures orthostatiques
(d) mise en place du pied de l'utilisateur dans la chaussure pendant que l'utilisateur est assis
(e) palpation de l'articulation sous-talaire de l'utilisateur pour l'amener à une position neutre
(f) l'utilisateur se tenant debout en faisant reposer un poids égal sur chaque pied tout en maintenant la position neutre de l'articulation sous-talaire, pression vers l'intérieur sur les bords médians et latéraux de la chaussure de manière à conformer le dispositif orthostatique maintenant moulable à la position neutre du pied de l'utilisateur, et
(g) refroidissement du dispositif.

2. Procédé selon la Revendication 1, dans lequel le courant d'air chauffé est appliqué en un cycle séquentiel d'environ 5 secondes de mode actif suivies d'environ 5 secondes de mode inactif, le cycle étant répété jusqu'à ce que le dispositif ait subi un cycle de chauffage total d'environ 20 secondes.

3. Procédé selon la Revendication 1 ou la Revendication 2 dans lequel, après l'étape (f), des supports d'avant-pied et/ou d'arrière-pied sont appliqués au bord éloigné plantaire du dispositif.

4. Procédé selon l'une quelconque des Revendications 1 à 3, comprenant l'incorporation d'un insert amortisseur (5) dans le dispositif (1), adapté pour amortir les chocs dans la zone du pied d'un patient située immédiatement sous son calcanéum.

5. Procédé selon la Revendication 4, comprenant l'extension de la zone amortie dans une partie ou la totalité de l'épaisseur du dispositif (1).

6. Procédé selon l'une quelconque des Revendications 1 à 5, comprenant le revêtement de la surface supérieure dudit dispositif (1) avec une couche extérieure de type tissu (6).

7. Procédé selon l'une quelconque des Revendications 1 à 6, comprenant le formage du dispositif en acétate de vinyle éthylique.
